# EUROPEAN PATENT SPECIFICATION

(11) **EP 2 743 853 B1**
(45) Date of publication and mention of the grant of the patent: **28.10.2020**
(21) Application number: 13275309.6
(22) Date of filing: 11.12.2013
(51) Int. Cl.: G16B 30/00

(54) **Draw-ahead feature for biological sequence drawing applications**
Vorzeichnungsfunktion für Zeichnungsanwendungen biologischer Sequenzen
Caractéristique draw-ahead pour applications de dessin de séquence biologique

(30) Priority: 13.12.2012 US 201213714312
(43) Date of publication of application: 18.06.2014
(73) Proprietor: PerkinElmer Informatics, Inc., Waltham MA 02451 (US)
(72) Inventor: Smellie, Andrew, Candia, NH 03034 (US); Stapleton, Mike, Lexington, MA 02420 (US); Smith, Robin, Boston, MA 02114 (US)
(74) Representative: Wilson, Justin Scott

(56) References cited:
- EP-A1- 2 224 389
- Anonymous: "GENEINSPECTOR® 2.0 Tutorials & User Manual", Textco BioSoftware, Inc. , 30 April 2012 (2012-04-30), pages 89-110, XP002770286, Retrieved from the Internet: URL:http://www.textco.com/files/manuals/GI _2.0_Manual.pdf [retrieved on 2017-05-17]

## Description

### Background

Biological sequence and large molecule rendering software is widely used by research and educational institutions to depict biological sequences, including nucleotide and amino acid sequences of interest. Such large molecules can be graphically represented in various ways; for example, a biological sequence can be represented textually by a sequence of letters corresponding to nucleotide codes or amino acid codes. Alternatively, a two- or three-dimensional graphical representation may be determined from a nucleotide and/or amino acid sequence to depict the arrangements of atoms, nucleotides, and/or amino acid residues of the biological molecule as a chemical structure, a ball-and-stick model, a ribbon diagram, a space-filling model, or an electrostatic model.

Current methods for drawing and editing sequence data and the resulting structural model on a computer utilize mouse-driven or touch pad commands that include pointing and clicking on displayed menu items in a graphical user interface. Existing biological sequence rendering applications for handheld electronic devices such as tablet computers and portable phones utilize the same menu-driven paradigm. These applications can be clumsy when attempting to draw biological sequences including many separate elements. For example, entering or editing large detailed sequences can be painstaking, tedious, and prone to error.

### Summary of the Invention

Described herein are various embodiments of systems, methods, and apparatus that allow a user to electronically draw and edit a biological sequence and its resulting structure. By offering a user pre-selected portions of a biological sequence in a user-friendly, intuitive way, the systems, methods, and apparatus described herein provide efficient and accurate tools for drawing and editing biological sequences.

In various embodiments, the systems, methods, and apparatus utilize or include a tablet computer, a mobile phone device, or any other computer device or system capable of receiving input. The systems, methods, and apparatus have applications in a wide variety of industries that create and edit biological sequences, such as the reagent industry, the publishing industry, and/or the web search industry.

Elements of embodiments described with respect to a given aspect of the invention may be used in various embodiments of another aspect of the invention. Multiple features may be combined together provided that the combined features are not technically incompatible with one another. For example, it is contemplated that features of dependent claims depending from one independent claim can be used in apparatus, articles, systems, and/or methods of any of the other independent claims or dependent claims.

In one aspect of the present disclosure, an apparatus for creating a representation of an in-progress biological sequence includes a memory for storing a set of instructions and a processor for executing the set of instructions, where the instructions, when executed, cause the processor to provide a representation of at least a portion of the in-progress biological sequence for presentation on a graphical display, and receive an input corresponding to an amendment to the portion of the in-progress biological sequence. The instructions cause the processor to identify, based at least in part upon a partial match with the amended in-progress biological sequence, one or more biological sequence scaffold candidates selected from an active database of biological sequence scaffold candidates, wherein the one or more biological sequence scaffold candidates comprises a minimum of at least 2 amino acids or at least 10 nucleotides. The instructions cause the processor to provide the one or more biological sequence scaffold candidates for presentation on the graphical display as option(s) for selection by a user for the amendment. The instructions also cause the processor to receive an indication of user selection of a first biological sequence scaffold candidate of the one or more biological sequence scaffold candidates provided, and append the first biological sequence scaffold candidate to the portion of the in-progress biological sequence or replace or partially replace the portion of the in-progress biological sequence with the first biological scaffold candidate, causing the regeneration of the graphical display to include the first biological sequence scaffold candidate in the in-progress biological sequence.

In some embodiments, the instructions, when executed, further cause the processor to, prior to providing the one or more biological sequence scaffold candidates for presentation, determine that a total number of the one or more biological sequence scaffold candidates does not exceed a threshold number of biological sequence scaffold candidates.

In some embodiments, the one or more biological sequence scaffold candidates include one or more commonly used biological sequence scaffold candidates. The commonly used biological sequence scaffold candidates may be scaffolds input and/or selected by a user or group of users at least a threshold number of times.

In some embodiments, the instructions, when executed, cause the processor to arrange the one or more biological sequence scaffold candidates in a ranked order prior to providing the one or more biological sequence scaffold candidates for presentation on the graphical display. Arranging the one or more biological sequence scaffold candidates in the ranked order may include identifying a usage count associated with each biological sequence scaffold candidate of the one or more biological sequence scaffold candidates. Arranging the one or more biological sequence scaffold candidates in the ranked order may include matching a user identifier associated with at least one biological sequence scaffold candidate of the one or more biological sequence scaffold candidates to a user identifier associated with the portion of the biological sequence.

In some embodiments, receiving the input includes receiving the input, over a network, from a computing device. The one or more biological sequence scaffold candidates may be stored in the memory.

The one or more biological sequence scaffold candidates include at least 10 nucleotides or at least 2 amino acids. The one or more biological sequence scaffold candidates may include about 25, 50, 75, 100, 150, 200, or 300 nucleotides. The one or more biological sequence scaffold candidates may include about 10, 20, 40, 60, 80, or 100 amino acid residues.

In one aspect of the present disclosure, a non-transitory computer readable medium has instructions stored thereon that, when executed, cause a processor to provide a representation of at least a portion of an in-progress biological sequence for presentation on a graphical display, and receive an input corresponding to an amendment to the portion of the in-progress biological sequence. The instructions, when executed, cause the processor to identify, based at least in part upon a partial match with the amended in-progress biological sequence, one or more biological sequence scaffold candidates selected from an active database of biological sequence scaffold candidates, wherein the one or more biological sequence candidates comprises a minimum of at least 2 amino acids or at least 10. The instructions, when executed, cause the processor to provide the one or more biological sequence scaffold candidates for presentation on the graphical display as option(s) for selection by a user for the amendment. The instructions also cause the processor to receive an indication of user selection of a first biological sequence scaffold candidate of the one or more biological sequence scaffold candidates provided, and append the first biological sequence scaffold candidate to the portion of the in-progress biological sequence or replace or partially replace the portion of the in-progress biological sequence with the first biological scaffold candidate, causing the regeneration of the graphical display to include the first biological sequence scaffold candidate in the in-progress biological sequence.

In some embodiments, a portion of the in-progress biological sequence is all of the in-progress biological sequence.

In one aspect of the present disclosure, a method of creating a representation of an in-progress biological sequence includes providing a representation of at least a portion of the in-progress biological sequence for presentation on a graphical display of a user computing device, and receiving an input corresponding to an amendment to the portion of the in-progress biological sequence. The method includes identifying, by a processor of a computing device, based at least in part upon a partial match with the amended in-progress biological sequence, one or more biological sequence scaffold candidates selected from an active database of biological sequence scaffold candidates, wherein the one or more biological sequence candidates comprises a minimum of at least 2 amino acids or at least 10 nucleotides. The method includes providing the one or more biological sequence scaffold candidates for presentation on the graphical display as option(s) for selection by a user for the amendment. The method also includes receiving, by the processor, an indication of user selection of a first biological sequence scaffold candidate of the one or more biological sequence scaffold candidates provided, and appending, by the processor, the first biological sequence scaffold candidate to the portion of the in-progress biological sequence or replace or partially replace the portion of the in-progress biological sequence with the first biological scaffold candidate, causing the regeneration of the graphical display to include the first biological sequence scaffold candidate in the in-progress biological sequence.

In some embodiments, the user computing device is the computing device. The amendment may include addition of at least one of a nucleotide or amino acid residue. The amendment may include removal of at least one of a nucleotide or amino acid residue.

### Brief Description of the Drawings

The foregoing and other objects, aspects, features, and advantages of the invention will become more apparent and may be better understood by referring to the following description taken in conjunction with the accompanying drawings, in which:
FIG. 1 is an example screen shot depicting a utility for creating or editing a representation of a biological sequence;
FIGS. 2A and 2B illustrate biological sequence identification from a portion of a representation of a biological sequence;
FIG. 3 is a flow chart of an example method for identifying and storing biological sequence scaffold candidates for use in a draw-ahead functionality of a utility for representation of a biological sequence;
FIGS. 4A and 4B illustrate example screen shots depicting a biological sequence scaffold candidate being presented to a user as a draw-ahead option based upon a portion of a representation of a biological sequence;
FIG. 5 is a flow chart of an example method for identifying and presenting biological sequence scaffold candidates in a draw-ahead functionality of a utility for representation of a biological sequence;
FIG. 6 is a schematic diagram of an example system for drawing or editing biological sequences;
FIG. 7 is a block diagram of an example computing device and an example mobile computing device.

The features and advantages of the present disclosure will become more apparent from the detailed description set forth below when taken in conjunction with the drawings, in which like reference characters identify corresponding elements throughout. In the drawings, like reference numbers generally indicate identical, functionally similar, and/or structurally similar elements.

### Description

It is contemplated that apparatus, systems, and methods of the claimed invention encompass variations and adaptations developed using information from the embodiments described herein. Adaptation and/or modification of the apparatus, systems, and methods described herein may be performed by those of ordinary skill in the relevant art.

Throughout the description, where apparatus and systems are described as having, including, or comprising specific components, or where processes and methods are described as having, including, or comprising specific steps, it is contemplated that, additionally, there are apparatus and systems of the present invention that consist essentially of, or consist of, the recited components, and that there are processes and methods according to the present invention that consist essentially of, or consist of, the recited processing steps.

It should be understood that the order of steps or order for performing certain actions is immaterial so long as the invention remains operable. Moreover, two or more steps or actions may be conducted simultaneously.

In general, in various embodiments, the present invention pertains to apparatus, systems, and methods for drawing biological sequences on a computing device. The computing device may be, for example, a personal computer, a workstation, a tablet computer (e.g., an Apple® IPad® by Apple Inc. of Cupertino, CA), or a mobile phone device. As used herein, the term "biological sequence" refers to the sequence of nucleotide or amino acid residues of a biological molecule (e.g., a DNA molecule, an RNA molecule, or a polypeptide). A biological sequence can be graphically represented in various ways, e.g., textually by a sequence of letters (e.g., using a 1-letter nucleotide code or using a 1-letter or 3-letter amino acid code), or structurally (e.g., as a chemical structure, a ball-and-stick model, a ribbon diagram, a space-filling model, or an electrostatic model). Structural representations can be two-dimensional or three-dimensional. In certain embodiments, textual representations can be organized in groups of a certain number of nucleotide or amino acid codes, for example, in a series of groups of 10 codes each.

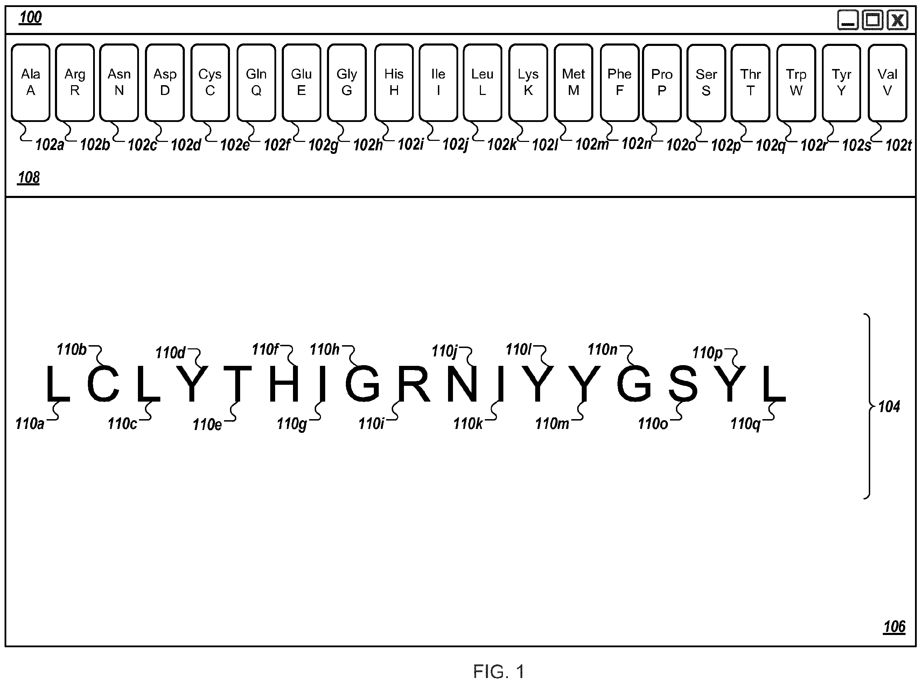

Referring to FIG. 1, a screen shot illustrates an example user interface 100 of a utility for creating and/or editing a representation of a biological sequence. The user interface 100 includes a series of editing tools 102 for building a representation of a biological sequence, e.g., a biological sequence 104 presented within an editing pane 106. A user, in some implementations, may select one of the editing tools 102 representing a nucleotide, a modified nucleotide, an amino acid residue, or a modified amino acid residue, in order to place the selected nucleotide, modified nucleotide, amino acid residue, or modified amino acid residue into the biological sequence 104. For example, the user may click on one of the editing tools 102 and/or drag and drop the selected feature into the editing pane 106 to add the selected feature at a desired position within or on the current biological sequence 104. Alternatively, or additionally, in some embodiments, the user may type letters corresponding to nucleotide or amino acid codes to enter the sequence into the editing pane.

In some implementations, a user may edit the biological sequence 104 by selecting one or more amino acid residues 110 in the biological sequence 104. The amino acid residue 110, in some examples, may be selected by clicking on an appropriate location of the biological sequence 104 with a mouse or other user interface device or by delivering a tap gesture upon a touch screen interface at the appropriate location of the biological sequence 104 which is to be amended. The user may then modify the biological sequence 104 at the selected amino acid residue 110 by selecting one of the editing tools 102 from a menu 108. For example, the user may select an amino acid residue 110h within the biological sequence 104, then select a replacement amino acid residue (e.g., 102f) by tapping the user interface 100 at the location of the corresponding tool 102f. In other examples, the user may edit or add to the biological sequence 104 a group of amino acid residues (e.g., a consensus sequence and/or a peptide sequence). Additionally, the drawing/editing utility may determine whether or not a given edit would result in a biological sequence that has a desired feature (e.g., is hydrophobic or hydrophilic) and may limit executable edits to only those resulting in biological sequences having the desired feature.

Although FIG. 1 depicts an exemplary screen shot illustrating a user interface of a utility for creating and/or editing an amino acid sequence, the present disclosure includes methods, systems, and apparatuses for creating and/or editing nucleic acid sequences (e.g., DNA or RNA sequences) and hybrid sequences (e.g., containing nucleic acid sequences and amino acid sequences).

In some implementations, based upon a representation of a biological sequence or a portion of a representation of a biological sequence (e.g., a biological sequence in progress), a biological sequence fragment matching utility identifies one or more portions of the representation of a biological sequence (e.g., as saved to a system via the biological sequence drawing utility) for presentation later as selectable biological sequence scaffolds to use when building or otherwise editing a representation of a biological sequence. In this manner, for example, a user may be provided the opportunity to reuse portions of a current drawing or a former drawing when building or otherwise editing a representation of a biological sequence, thereby saving time and preserving accuracy. In some implementations, biological sequence scaffolds derived from one or more representations of biological sequences created by other users of the system may be presented as fragments (e.g., biological sequence scaffolds) to a particular user for building or otherwise editing a representation of a biological sequence.

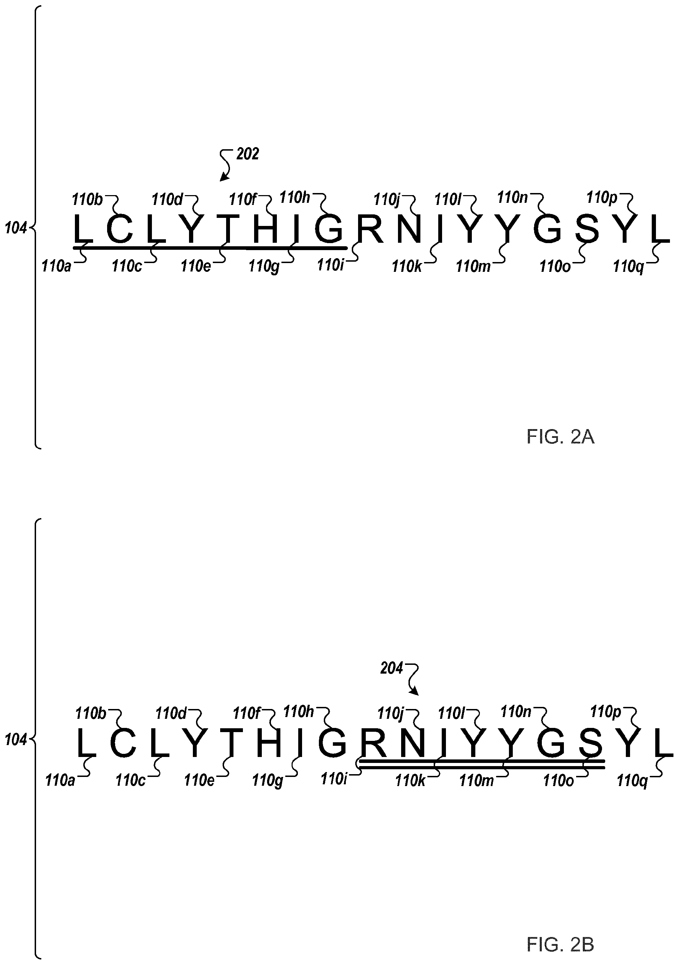

For example, turning to FIGS. 2A and 2B, an example of two different biological sequence scaffolds 202, 204 are illustrated in relation to the biological sequence 104. Each biological sequence scaffold 202, 204, for example, represents a fragment or portion of a representation of a biological sequence. The biological sequence scaffolds 202, 204, in some implementations, include one or more nucleotide, one or more modified nucleotide, one or more amino acid residues, one or more modified amino acid residues, or combinations thereof. In some implementations, the biological sequence scaffolds 202, 204 include one or more consensus sequences and/or peptide sequences.

Turning to FIG. 2A, a first biological sequence scaffold 202 is identified by single underlining. The first biological sequence scaffold 202, for example, includes amino acid residues 110a, 110b, 110c, 110d, 110e, 110f, 110g, and 110h.

Turning to FIG. 2B, a second biological sequence scaffold 204 is identified by double underlining. The second biological sequence scaffold 204, for example, includes amino acid residues 102i, 102j, 102k, 1021, 102m, 102n, and 102o. Note that amino acid residues 102p and 102q are not included as part of either the first biological sequence scaffold 202 or the second biological sequence scaffold 204. In some implementations, one or more portions of a biological sequence may be pruned from the biological sequence prior to identifying a biological sequence scaffold candidate.

In the example shown in FIG. 2A and 2B, the biological sequence scaffold 204 is identified as a partner biological sequence scaffold to the biological sequence scaffold 202. For example, in a different drawing of a biological sequence, upon identification of the biological sequence scaffold 202 in that biological sequence, it may be assumed that it is likely that additional amino acid residues (e.g., yet to be drawn) may comprise the amino acid residues of the biological sequence scaffold 204. In this manner, a draw-ahead utility may identify the first biological sequence scaffold 202 within a new representation of a biological sequence and, in response, offer the second biological sequence scaffold 204 as a continuation of the drawing in progress.

In some implementations, the combination of two biological sequence scaffolds (e.g., biological sequence scaffold 202 and biological sequence scaffold 204) may be identified as a separate biological sequence scaffold candidate (e.g., a "super biological sequence scaffold candidate") combining two smaller biological sequence scaffold candidates. For example, within a representation of a large biological sequence, varying sizes of sub-sequences may be identified as fragments which are likely to reoccur in representation of different biological sequences.

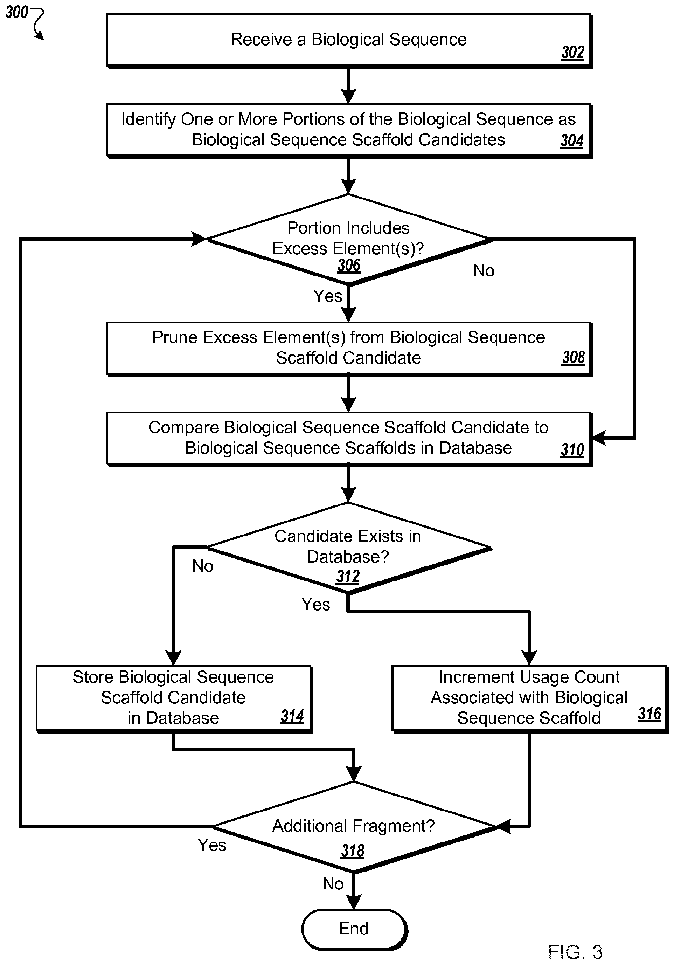

FIG. 3 is a flow chart of an example method 300 for identifying and storing biological sequence scaffolds for use in a draw-ahead utility of a biological sequence editing application. For example, the biological sequence scaffolds may be identified within prior (or current) drawings created by a particular user or from drawings created by a number of users. In some implementations, the method 300 may be used to mine the drawings created by a number of users accessing a common software license or storing drawings to a common repository (e.g., networked storage device). The method 300, for example, may be used in identifying the first biological sequence scaffold 202 and the second biological sequence scaffold 204, shown in FIGS. 2A and 2B.

In some implementations, the method 300 begins with receiving a representation of at least a portion of a biological sequence (302). The representation of a biological sequence, in some examples, may include a biological sequence drawing-in-progress, a complete representation of a biological sequence, a representation of a biological sequence imported from a separate software application, or a biological sequence representation stored within a document repository.

In some implementations, one or more portions of the biological sequence are identified as biological sequence scaffold candidates (304). In some implementations, a biological sequence scaffold candidate may include a minimum number of elements (e.g., biological sequence features) such as, for example, at least 2, 5, 10, 20, 30, 40, 50, 60, 70, 80, 90, 100, or more, amino acid residues. In some implementations, a biological sequence scaffold candidate may include at least 10, 25, 50, 75, 100, 125, 150, 175, 200, 225, 250, 275, 300, 350, 400, 450, 500, or more, nucleotides. A biological sequence scaffold, in some implementations, is composed of or comprises amino acid residues. In some implementations, a biological sequence scaffold may be identified by receiving a biological sequence as a "favorite" from a user, for example, through a user interface capture feature. For example, a user may enter or select one or more biological sequence scaffolds that the user anticipates drawing frequently.

In some implementations, a collection of biological sequences are received and reviewed to identify biological sequence scaffold candidates. For example, biological sequences may be captured in a registration system (e.g., registering biological sequences to associate with a software license or user identification within a system including a chemical formula and/or biological sequence drawing program with draw-ahead feature), culled from public data sets, read from a database such as Genbank or UniProt, captured as new biological sequences from an electronic lab notebook (ELN) system, or identified through optical character recognition (OCR) systems.

In reviewing a collection of biological sequences (e.g., as obtained from one or more of the avenues identified above), in some implementations, a routine is used to identify one or more maximum common subsequences among the collection (e.g., appearing two or more times, etc.). In some implementations, a routine iteratively identifies common subsequences within the collection. The routine, for example, may identify common subsequences including at least a threshold number of elements (e.g., biological sequence features such as consensus sequences), as described above. The routine may identify the largest common subsequence among a collection of biological sequences.

If a potential biological sequence scaffold candidate includes one or more excess elements (306), in some implementations, the portion may be pruned to determine a biological sequence scaffold candidate (308). For example, as illustrated within FIG. 2B, the amino acid residues 102p and 102q may be pruned away. In other words, in this example, elements not belonging to a biological sequence scaffold are eligible for pruning. In some implementations, modified nucleotides and/or modified amino acid residues may be pruned when identifying a biological sequence scaffold candidate. In other examples, modified nucleotides and/or modified amino acid residues may be replaced with naturally existing nucleotides and/or naturally existing amino acid residues.

In some implementations, the biological sequence scaffold candidate is compared to biological sequence scaffolds in a database of biological sequence scaffolds (310). In some implementations, a biological sequence creation and editing application includes a database of common biological sequence scaffolds. For example, upon installing the application, a draw-ahead feature may have access to a database of common biological sequence scaffolds for presentation as draw-ahead options. The database of biological sequence scaffolds, in some implementations, contains one or more biological sequence scaffolds identified as portions of other representations of biological sequences. For example, the method 300 may have been performed previously on a biological sequence to derive one or more biological sequence scaffolds.

In some implementations, a sub-sequence of a biological sequence scaffold candidate may be compared to a sub-sequence of a biological sequence scaffold within the database. For example, as illustrated in FIGS. 2A and 2B, the first biological sequence scaffold 202 may be compared to a first portion of the candidate biological sequence scaffold. Features of the candidate such as, in some examples, the number and placement of amino acid residues or the placement of particular amino acid residues may be compared to one or more biological sequence scaffolds in the database to identify a match.

If a match of the biological sequence scaffold candidate is identified within the database (312), in some implementations, a usage count associated with the identified biological sequence scaffold is incremented (316). For example, to track a relative popularity of a particular biological sequence scaffold, in some implementations, the number of times the particular biological sequence scaffold has been identified in a representation of a biological sequence may be tracked. In some implementations, the usage count identifies, in part, the number of times a particular biological sequence scaffold has been selected when presented as a draw-ahead candidate by a draw-ahead feature.

If, instead, a match is not found in relation to the biological sequence scaffold candidate, in some implementations, the biological sequence scaffold candidate is stored in the database (314). In some implementations, a foundational portion of the biological sequence scaffold candidate may be identified such that, in response to matching the foundational portion with a portion of a representation of a biological sequence, the remainder of the biological sequence scaffold may be offered as a biological sequence scaffold candidate for draw-ahead purposes. For example, a first set of two or more amino acid residues may be identified as a foundational portion of the biological sequence scaffold. In some implementations, the method 300 repeats the pruning and comparison steps for the remaining biological sequence scaffold candidates (318).

Although the method 300 has been described in relation to a series of steps performed in an example order, in other implementations, one or more of the steps of the method 300 may be performed in a different order and/or in parallel, and one or more steps may be added to the method 300. Furthermore, one or more of the steps of the method 300, in other implementations, may be combined or removed. For example, in some implementations, two or more previously iterated biological sequence scaffold candidates may be combined as a "super biological sequence scaffold candidate", where the "super biological sequence scaffold candidate" may be compared to the biological sequence scaffolds identified by the database. In another example, in some implementations, each biological sequence scaffold may be associated with one or more users (e.g., user identifiers associated with drawings containing the particular biological sequence scaffold. For example, should user "Bob" commonly draw biological sequence scaffold A, a usage count associated with both biological sequence scaffold A and user Bob may be incremented, such that biological sequence scaffold A will be promoted in priority in relation to other biological sequence scaffold candidates when identifying two or more draw-ahead options for Bob. Further to the example, user "Gary," who has never used biological sequence scaffold A (although it has been used repeatedly by Bob), may be offered a different biological sequence scaffold as a primary candidate for draw-ahead purposes (e.g., a biological sequence scaffold previously used by Gary) even when drawing a same biological sequence. Other modifications of the method 300 are possible without straying from the intent and purpose of the method 300.

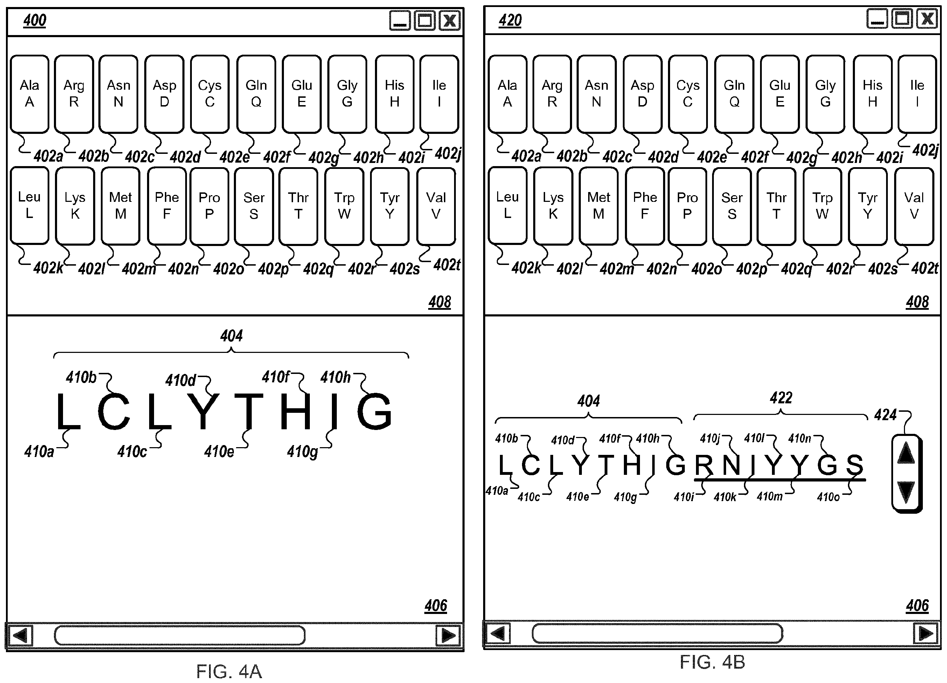

FIGS. 4A and 4B illustrate example screen shots depicting a biological sequence scaffold candidate 422 being presented to a user as a draw-ahead option based upon a portion 404 of a representation of a biological sequence. The draw-ahead option, for example, may have been previously identified within a different representation of a biological sequence, for example as described by the method 300 illustrated in FIG. 3.

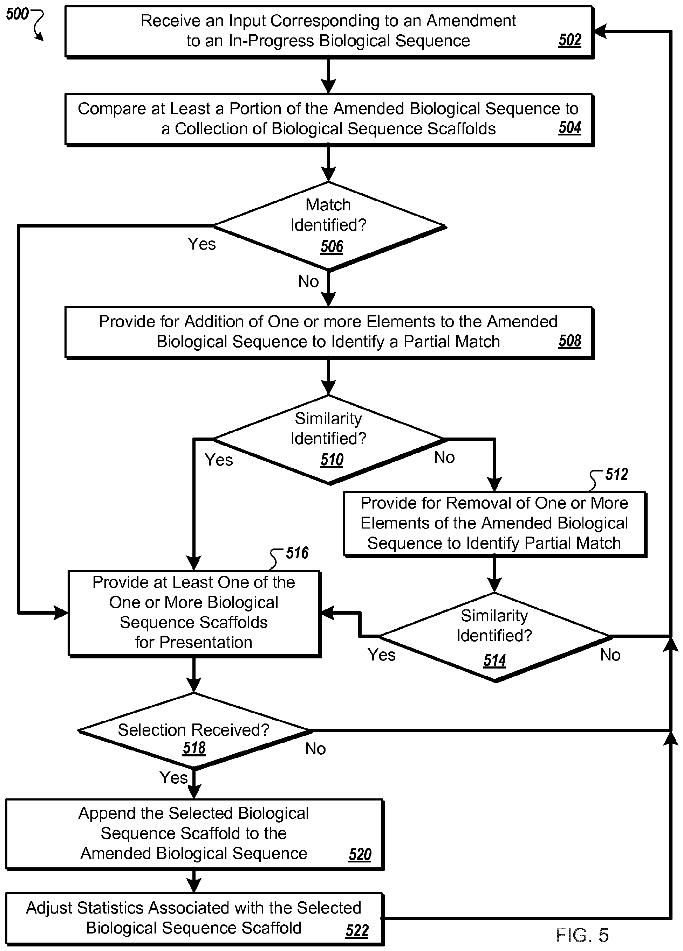

Turning to FIG. 4A, in a first screen shot 400, a user is working on a representation of a biological sequence, currently containing the portion 404 including a set of amino acid residues 410a, 410b, 410c, 410d, 410e, 410f, 410g, and 401h. In some implementations, the user constructed the portion 404 using a set of editing tools 402, as illustrated within a tool menu 408. For example, a user may select a particular editing tool 402 from the menu, then select a location in an editing pane 406 for positioning of the selected editing tool feature. In another example, the user may drag and drop a particular editing tool feature 402 into the editing pane 406. In another example, the user may input the particular editing tool feature 402 using a keyboard by striking a particular key associated with the particular feature 402. Upon each addition of an element of the representation of the biological sequence, in some implementations, a draw-ahead utility invokes a matching function to identify one or more biological sequence scaffold candidates to present in relation to the existing portion 404 of a biological sequence. An example of a method for identifying a biological sequence scaffold candidate based upon a portion of a representation of a biological sequence is described in relation to FIG. 5.

Turning to FIG. 4B, in a second screen shot 420, the biological sequence scaffold candidate 422 is illustrated as an extension of the existing portion 404. In some implementations, the biological sequence scaffold candidate 422 is visually rendered in a manner that differentiates the amino acid residues of the biological sequence scaffold candidate 422 from the amino acid residues of the existing portion 404. For example, as illustrated, the biological sequence scaffold candidate 422 is rendered in part using underlining. In other examples, the biological sequence scaffold candidate 422 may be rendered as a semi-opaque image, in a different color, partially removed from the existing portion 404 (e.g., like a puzzle piece that could be pulled into position), highlighted, outlined, and/or filled in a different color, and/or in a different font. In some implementations, a second (e.g., preview) pane may pop-up, overlay, or be rendered within the editing pane 406, where the preview pane may illustrate the addition of the biological sequence scaffold candidate 422 to the existing portion 404.

In some implementations, based upon a partial match of an existing portion of a representation of a biological sequence, one or more elements (e.g., amino acid residues) may be added to the existing portion during presentation of a biological sequence scaffold candidate.

Next to the biological sequence scaffold candidate 422, as illustrated in FIG. 4B, a selection control 424, when selected, may present additional biological sequence scaffold candidates. For example, by toggling up or down using the directional arrows of the selection control 424, the user may be presented with one or more additional biological sequence scaffold candidates. In some implementations, activation of the selection control 424 causes the replacement of the biological sequence scaffold candidate 422 (and, optionally, any elements added to the existing portion 404 to match the biological sequence scaffold candidate 422 to the existing portion 404) with a second biological sequence scaffold candidate (and, optionally, any new elements that may be added to the existing portion 404 to match the second biological sequence scaffold candidate to the existing portion 404). In other implementations, activation of the selection control 424 may launch a preview window of biological sequence scaffold sequences, such that a user may scroll through and select a particular biological sequence scaffold candidate for presentation in relation to the existing portion 404. Although illustrated as a bidirectional toggle control, other controls are possible.

Additionally, in some implementations, a natural language interface may be used to provide input to the selection of biological sequence scaffold candidates. For example, the terms "next" and "back", when uttered, may cause the user interface to scroll through biological sequence scaffold candidates.

Once a biological sequence scaffold candidate has been decided upon, in some implementations, the selection control 424 is removed from the editing pane and the biological sequence scaffold candidate is presented in the same drawing style (e.g., color, line width, transparency, background, etc.) as the existing portion 404. In some implementations, a user may select the presented biological sequence scaffold candidate 422 (e.g., touching, clicking, mousing over while activating an enter key, etc.) to indicate acceptance of the biological sequence scaffold candidate 422. In other implementations (not illustrated), a portion of the selection control 424 or a separate control may be used to indicate acceptance of the biological sequence scaffold candidate 422. In implementations involving a natural language interface, for example, a term such as "select" or "add", when uttered, may indicate acceptance of an active (e.g., currently presented) biological sequence scaffold candidate.

FIG. 5 is a flow chart of an example method 500 for identifying and presenting biological sequence scaffold candidates in a draw-ahead functionality of a utility for representation of a biological sequence. The method 500, in some implementations, may be used to identify and present one or more biological sequence scaffold candidates based upon an existing portion of a representation of a biological sequence. As illustrated in FIGS. 4A and 4B, for example, the method 500 may review the existing portion 404 and offer the biological sequence scaffold candidate 422 as an option for continuing the drawing of the representation of a biological sequence.

The method 500, in some implementations, begins with receiving an input corresponding to an amendment to a representation of a biological sequence (502). The representation of a biological sequence, for example, includes one or more nucleotides, modified nucleotides, amino acid residues, or modified amino acid residues. The representation of the biological sequence, for example, may have been developed by a user "from scratch", e.g., through adding elements (e.g., nucleotides and/or amino acid residues) to a work area of a biological sequence drawing tool. In another example, the representation of the biological sequence may be brought into the biological sequence drawing tool (e.g., selected from a favorites list, opened from a saved file, imported from an electronic lab notebook, etc.). The representation of the biological sequence, in some implementations, includes a portion or a fragment of a gene or a polypeptide. A user, interacting with a representation of a biological sequence within a graphical user interface, may make a modification to the representation of the biological sequence such as, in some examples, addition of a nucleotide or amino acid residue, and/or removal of a nucleotide or amino acid residue. In some implementations, the method 500 may only be invoked based upon particular types of amendments. For example, in some examples, the method 500 may be invoked in response to addition and/or deletion of a group of nucleotides and/or amino acid residues having at least a minimum specified size, in response to a user saving a current state of a draft biological sequence, or in response to the addition of a biological sequence scaffold candidate to the representation of a biological sequence. As used herein, an "amendment" of a biological sequence may include modification of, as well as creation of, a biological sequence.

In some implementations, at least a portion of the amended biological sequence is compared to a collection of biological sequence scaffold candidates (504). The collection of biological sequence scaffold candidates, in some implementations, includes one or more previously drawn biological sequences or portions of biological sequences. In some implementations, the collection of biological sequence scaffold candidates includes one or more commonly identified portions of biological sequences, for example as identified through a database of graphical representations of known biological sequences. In a particular example, a collection of known biological sequences, such as Genbank or UniProt, can be obtained to identify commonly reoccurring biological sequences, or fragments thereof, as biological sequence scaffold candidates. In another example, an entity (e.g., university, corporation, research organization, etc.) may populate the database of biological sequence scaffold candidates with one or more biological sequence scaffolds (e.g., imported from a database of biological sequences previously constructed by the entity). The biological sequence scaffold candidates, in some implementations, are categorized and stored in a database for querying based upon a partial match (e.g., a match of 2, 5, 10, 20, 30, 40, 50, 60, 70, 80, 90, 100, or more nucleotides and/or amino acid residues) between the reoccurring biological sequence or fragment thereof and a portion of a representation of a biological sequence.

The portion of the amended biological sequence to be compared to the collection of biological sequence scaffold candidates may be identified using various methods. For example, the utility may identify the location of the user's edit of the biological sequence. Then, the utility may look in the immediate locale to find fragments (biological sequence scaffold candidates) in the database that have a subsequence in common with this locale. The search may be biased by biological sequence scaffold candidates that are drawn more often or more frequently than others.

Thus, in some implementations, common biological sequence fragments identified within the collection of biological sequence scaffold candidates may be used to provide guidelines on how to break down an amended biological sequence into one or more portions for purposes of identifying a matching biological sequence scaffold candidate from the collection. For example, frequently occurring sub-sequences of the biological sequence scaffold candidates in the collection may be identified, and these frequently occurring sub-sequences may be used as a basis for breaking down a representation of a biological sequence into a series of portions, or fragments. In some implementations, a modified section of the biological sequence, such as two or more amino acid residues added to the biological sequence, is compared to the collection of biological sequence scaffold candidates. For example, the portion may be selected to contain at least a threshold number of elements, such as at least 2, 5, 10, 20, 30, 40, 50, 60, 70, 80, 90, 100, or more nucleotides and/or amino acid residues.

In some implementations, a biological sequence scaffold candidate may be identified based upon nucleotide and/or amino acid sequence similarity, e.g., as represented by a biological sequence similarity measure such as using a known algorithm (e.g., BLAST or FASTA).

If no matches or similarities are located (514), in some implementations, the method 500 returns to waiting to receive an input corresponding to an amendment to the representation of the biological sequence (502). Then, based upon further input, such as addition or removal of another nucleotide and/or amino acid residue to or from the representation of the biological sequence, the method 500 may locate a biological sequence scaffold candidate for appending to or otherwise modifying the representation of the biological sequence.

Conversely, if any match or similarity is located, in some implementations, the matching and/or similar biological sequence scaffold candidates are provided for presentation within a graphical user interface (516). For example, the potential match may be presented as a selectable amendment in any of the manners described in relation to FIG. 4B.

If a selection of a particular biological sequence scaffold candidate of the one or more biological sequence scaffold candidates is not received (518), in some implementations, the method 500 returns to waiting to receive an input corresponding to an amendment to the representation of the biological sequence (502).

If, instead, selection of a particular biological sequence scaffold candidate of the one or more biological sequence scaffold candidates is received (518), in some implementations, the selected biological sequence scaffold candidate is appended to the amended biological sequence (520), or the in-progress biological sequence is replaced or partially replaced with the selected biological sequence scaffold candidate, as appropriate. Appending the biological sequence scaffold candidate, for example, can include causing the re-generation of a graphical user interface to include the portion of the biological sequence scaffold candidate previously presented as an option to the user.

In some implementations, statistics associated with the selected biological sequence scaffold candidate are adjusted (522). To determine an order in which to present multiple biological sequence scaffold candidates to a user, the method 500 may adjust one or more statistics related to a selected biological sequence scaffold candidate. In some examples, the statistics may include a usage count, a timestamp, and a list of users who have selected the particular biological sequence scaffold candidate. The statistics may be gathered related to the biological sequence scaffold candidate in general and/or a per user basis. For example, a first user may more commonly draw a first set of biological sequence scaffold candidates, while a second user may more commonly draw a second set of biological sequence scaffold candidates. In some implementations, biological sequence scaffold candidates are collected as sub-portions, and statistics may be stored regarding particular combinations of sub-portions. For example, the method 500 may track the number of times the combination of the portion 404 without the amino acid residue 410a is used in combination with the portion 422, in comparison to the number of times the combination of the portion 404 in whole is used in combination with the portion 422. In some implementations, statistics may be collected related to the frequency in which two biological sequence scaffolds appear in a same biological sequence. For example, it may be determined that users who select (or draw) biological sequence scaffold Y are very likely to later select (or draw) biological sequence scaffold Z. Other statistics are possible.

Upon receipt of an additional amendment to the representation of the biological sequence (502), in some implementations, the method 500 may repeat.

Although the method 500 has been described in relation to a series of steps performed in an example order, in other implementations, one or more of the steps of the method 500 may be performed in a different order and/or in parallel, and one or more steps may be added to the method 500. For example, rather than providing one or more biological sequence scaffold candidates for presentation (516) after having only identified one or two matches (506), in some implementations, the method 500 may continue to attempt to identify similarities (e.g., as described in relation to steps 508 through 514) until a threshold number of matches (e.g., three, five, etc.) has been identified. Conversely, should identification of the one or more biological sequence scaffold candidates (506) produce greater than a threshold number of biological sequence scaffold candidates (e.g., five, ten, twenty, etc.), in some implementations, the method 500 prioritizes the matching biological sequence scaffold candidates in a ranked order and only provides the top N matches for presentation to the user (516). In other implementations, should identification of the one or more biological sequence scaffold candidates (506) produce greater than a threshold number of biological sequence scaffold candidates, the method 500 returns to waiting to receiving an additional amendment (502). For example, rather than overwhelm a user with constant options for biological sequence scaffold candidates, the method 500 may wait to present biological sequence scaffold candidates that may, presumably, have a higher chance of being desirable to the user. Prior to providing at least one of the one or more biological sequence scaffold candidates for presentation (516), in some implementations, the biological sequence scaffold candidates may be ranked in order of priority (e.g., according to one or more statistical values associated with the biological sequence scaffold candidates and/or according to whether a particular biological sequence scaffold candidate was a direct match or a similar match).

Furthermore, one or more of the steps of the method 500, in other implementations, may be combined or removed. For example, in some implementations, steps 512 and 514 may be removed, causing matches to only be served on the existing portion of the biological sequence. In some implementations, the method 500 may begin with identifying one or more biological sequence scaffold candidates for presentation to begin a drawing project of a new representation of a biological sequence. In one example, based upon user information (e.g., user preferences, user favorites list, user history, user group membership, etc.), the method 500 may identify one or more biological sequence scaffold candidates to present to the user as a basis for the new drawing project. Other modifications of the method 500 are possible without straying from the intent and purpose of the method 500.

In some implementations, systems, methods, and apparatus described herein include functionality for graphic representation of a biological sequence and/or a biological sequence scaffold candidate in various ways. For example, a biological sequence can be initially depicted textually using letters (e.g., a 1-letter nucleotide code and/or a 1-letter or 3-letter amino acid code). After initially inserting a portion of a biological sequence, a user can select to graphically represent the biological code structurally (e.g., as a chemical structure, a ball-and-stick model, a ribbon diagram, a space-filling model, or an electrostatic model). In some implementations, a user can depict a biological sequence scaffold candidate structurally, e.g., prior to or after selecting the biological sequence scaffold candidate to modify a biological sequence.

In some implementations, systems, methods, and apparatus described herein include functionality for additional sequence editing, as known in the art. For example, a biological sequence, or a fragment, can be analyzed for various physical characteristics, such as hydrophobicity, hydrophilicity, and predicted folding patterns (e.g., alpha helices or beta sheets). These physical characteristics can be graphically represented using the systems, methods, and apparatus described herein.

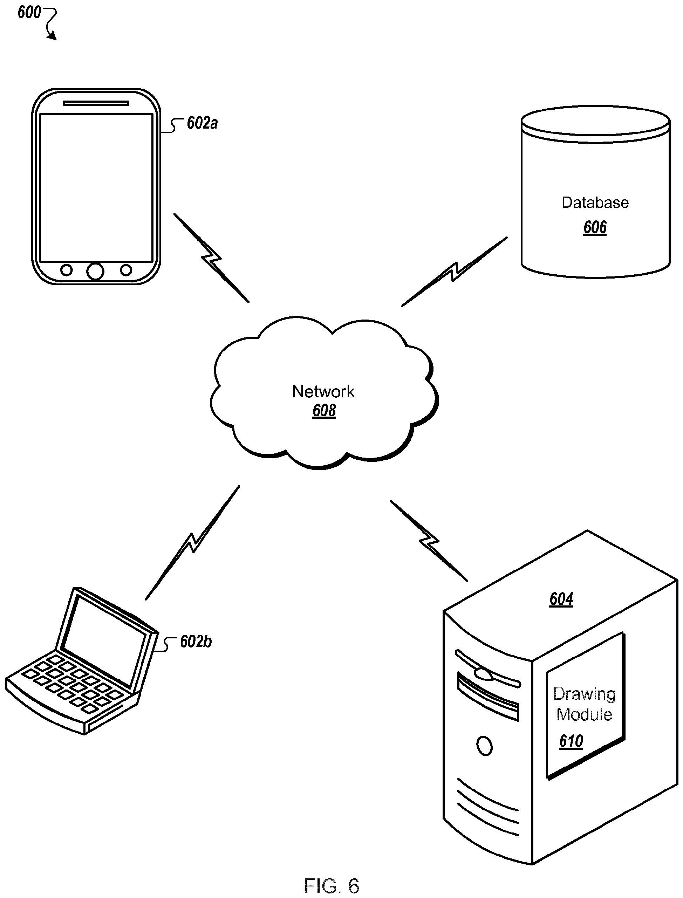

FIG. 6 depicts an example system 600 for drawing or editing representations of biological sequences. The system 600 includes client nodes 602a and 602b, a server node 604, a database 606, and, for enabling communications therebetween, a network 608. As illustrated, the server node 604 may include a drawing module 610.

The network 608 may be, for example, a local-area network (LAN), such as a company or laboratory Intranet, a metropolitan area network (MAN), or a wide area network (WAN), such as the Internet. Each of the client nodes 602, server node 604, and the database 606 may be connected to the network 608 through a variety of connections including, but not limited to, standard telephone lines, LAN or WAN links (e.g., T1, T3, 56 kb, X.25), broadband connections (e.g., ISDN, Frame Relay, ATM), or wireless connections. The connections, moreover, may be established using a variety of communication protocols (e.g., HTTP, TCP/IP, IPX, SPX, NetBIOS, NetBEUI, SMB, Ethernet, ARCNET, Fiber Distributed Data Interface (FDDI), RS232, IEEE 802.11, IEEE 802.11a, IEEE 802.11b, IEEE 802.11g, and direct asynchronous connections).

The client node 602a may be any type of wireless device, information appliance, tablet computer, personal digital assistant, cellular phone, handheld device, or other portable computing device that is capable of both presenting information/data to, and receiving commands from, a user of the client node 602a (e.g., a molecular biologist). Similarly, the client node 602b may be any type of personal computer, Windows-based terminal, network computer, wireless device, information appliance, RISC Power PC, X-device, workstation, mini computer, main frame computer, set top box, or other computing device that is capable of both presenting information/data to, and receiving commands from, a user of the client node 602b. The client nodes 602 may include, for example, a graphical display device (e.g., a touch screen or a computer monitor), a data entry device (e.g., a keyboard, a touch screen, or a mouse pad), persistent and/or volatile storage (e.g., computer memory), and a processor. In one embodiment, the client node 602 includes a web browser, such as, for example, Internet Explorer® developed by Microsoft Corporation of Redmond, Washington, to connect to the World Wide Web.

For its part, the server node 604 may be any computing device that is capable of receiving information/data from and delivering information/data to the client nodes 602, for example over the network 608, and that is capable of querying, receiving information/data from, and delivering information/data to the server node 604. For example, as further explained below, the server node 604 may receive input (e.g., a multi-touch gesture) from a user of the client node 602, create or edit a biological sequence representation according to the input, and present or display the biological sequence representation to the user at the client node 602. The server node 604 may include a processor and persistent and/or volatile storage, such as computer memory.

The server node 604 may be any computing device that is capable of storing and managing collections of data, such as data relating to biological sequences.

As used herein, the term "server node" is broadly used to refer to any repository of information. The data stored within the server node 604 may be harvested from the server node 604 in any manner. In one embodiment, the harvesting is performed utilizing indexing and sequence recognition algorithms, and the harvested data is connected together by examining and correlating the disjointed information that is found.

The drawing module 610 of the server node 604 may be implemented as any software program and/or hardware device, for example an application specific integrated circuit (ASIC) or a field programmable gate array (FPGA), that is capable of providing the functionality described herein. It will be understood by one having ordinary skill in the art, however, that the illustrated module 610, and the organization of the server node 604, are conceptual, rather than explicit, requirements. For example, it should be understood that the drawing module 610 may in fact be implemented as multiple modules, such that the functions performed by the single module, as described herein, are in fact performed by the multiple modules.

Although not shown in FIG. 6, any or all of the client nodes 602, the server node 604, and the database 606 may also include its own transceiver (or separate receiver and transmitter) that is capable of receiving and transmitting communications, including requests, responses, and commands, such as, for example, inter-processor communications and networked communications. The transceivers (or separate receivers and transmitters) may each be implemented as a hardware device, or as a software module with a hardware interface.

It will also be understood by those skilled in the art that FIG. 6 is a simplified illustration of the system 600 and that it is depicted as such to facilitate the explanation of various embodiments of the present disclosure. For example, rather than being implemented on a single server node 604, the drawing module 610 may instead be implemented on a different computing device (not shown) and such computing devices may communicate with one another directly, over the network 608, or over another additional network (not shown). In yet another example, the functionality of the server node 604 may in fact be resident on the server node 604 (e.g., be implemented in the computer memory thereof). Additional options are for the server node 604 and/or the database 606 to be local to the client node 602 (such that they may all communicate directly without using the network 608), or for the functionality of the server node 604 and/or the database 606 to be implemented on the client node 602 (e.g., for the drawing module 610 and/or the server node 604 to reside on the client node 602). As such, the depiction of the system 600 in FIG. 6 is non-limiting.

In certain embodiments, the system 600 allows a user to draw and edit a biological sequence representation using one or more fingers on an input interface, such as a touch pad or touch screen, at the client tablet node 602a. The system 600, in some embodiments, allows a user to draw and edit a representation of a biological sequence using a mouse, stylus, keypad, trackball, or other input interface, such as an input interface at a client personal computer 602b. The input interface, in some implementations, may include a natural language processing module capable of converting utterances to a series of commands for activating controls of the user interface.

In general, the drawing module 610 in the server node 604 is configured to draw or revise the biological sequence representation according to the input from the user, as explained above with respect to the prior figures. The drawing module 610 may then provide an image (e.g., a collection of pixels) of the representation of the biological sequence for presentation to the user on the graphical display of the particular client node 602. Additionally, the drawing module 610 may present one or more biological sequence scaffold candidates for amendment to a representation of a biological sequence. The biological sequence scaffold candidate, for example, may be identified from biological sequence scaffold candidates stored within the database 606. In general, the system 600 may be used to perform any of the methods described herein.

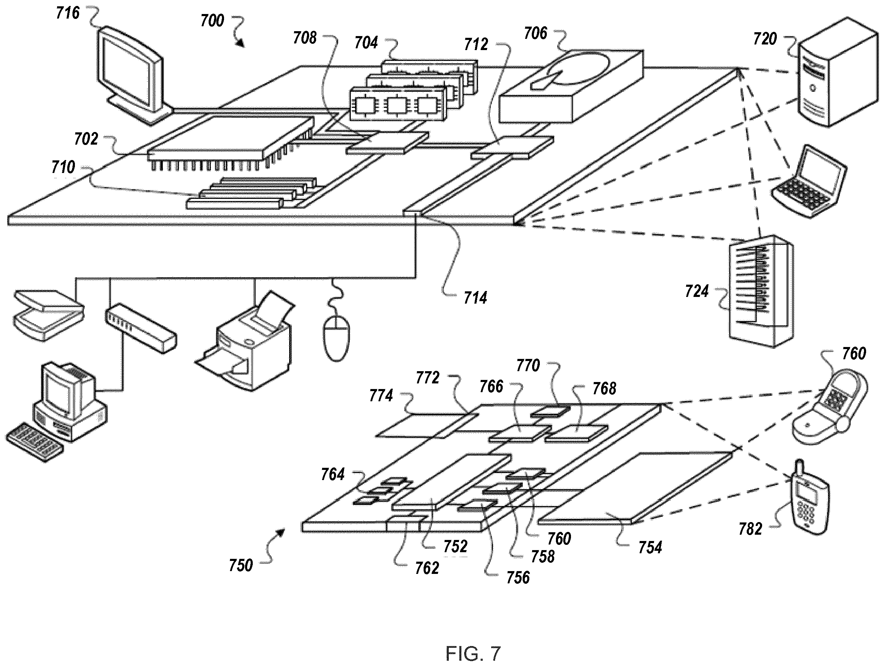

FIG. 7 shows an example of a computing device 700 and a mobile computing device 750 that can be used to implement the techniques described in this disclosure. The computing device 700 is intended to represent various forms of digital computers, such as laptops, desktops, workstations, personal digital assistants, servers, blade servers, mainframes, and other appropriate computers. The mobile computing device 750 is intended to represent various forms of mobile devices, such as personal digital assistants, cellular telephones, smart-phones, and other similar computing devices. The components shown here, their connections and relationships, and their functions, are meant to be examples only, and are not meant to be limiting.

The computing device 700 includes a processor 702, a memory 704, a storage device 706, a high-speed interface 708 connecting to the memory 704 and multiple high-speed expansion ports 710, and a low-speed interface 712 connecting to a low-speed expansion port 714 and the storage device 706. Each of the processor 702, the memory 704, the storage device 706, the high-speed interface 708, the high-speed expansion ports 710, and the low-speed interface 712, are interconnected using various busses, and may be mounted on a common motherboard or in other manners as appropriate. The processor 702 can process instructions for execution within the computing device 700, including instructions stored in the memory 704 or on the storage device 706 to display graphical information for a GUI on an external input/output device, such as a display 716 coupled to the high-speed interface 708. In other implementations, multiple processors and/or multiple buses may be used, as appropriate, along with multiple memories and types of memory. Also, multiple computing devices may be connected, with each device providing portions of the necessary operations (e.g., as a server bank, a group of blade servers, or a multi-processor system).

The memory 704 stores information within the computing device 700. In some implementations, the memory 704 is a volatile memory unit or units. In some implementations, the memory 704 is a non-volatile memory unit or units. The memory 704 may also be another form of computer-readable medium, such as a magnetic or optical disk.

The storage device 706 is capable of providing mass storage for the computing device 700. In some implementations, the storage device 706 may be or contain a computer-readable medium, such as a floppy disk device, a hard disk device, an optical disk device, or a tape device, a flash memory or other similar solid state memory device, or an array of devices, including devices in a storage area network or other configurations. Instructions can be stored in an information carrier. The instructions, when executed by one or more processing devices (for example, processor 702), perform one or more methods, such as those described above. The instructions can also be stored by one or more storage devices such as computer- or machine-readable mediums (for example, the memory 704, the storage device 706, or memory on the processor 702).

The high-speed interface 708 manages bandwidth-intensive operations for the computing device 700, while the low-speed interface 712 manages lower bandwidth-intensive operations. Such allocation of functions is an example only. In some implementations, the high-speed interface 708 is coupled to the memory 704, the display 716 (e.g., through a graphics processor or accelerator), and to the high-speed expansion ports 710, which may accept various expansion cards (not shown). In the implementation, the low-speed interface 712 is coupled to the storage device 706 and the low-speed expansion port 714. The low-speed expansion port 714, which may include various communication ports (e.g., USB, Bluetooth®, Ethernet, wireless Ethernet) may be coupled to one or more input/output devices, such as a keyboard, a pointing device, a scanner, or a networking device such as a switch or router, e.g., through a network adapter.

The computing device 700 may be implemented in a number of different forms, as shown in the figure. For example, it may be implemented as a standard server 720, or multiple times in a group of such servers. In addition, it may be implemented in a personal computer such as a laptop computer 722. It may also be implemented as part of a rack server system 724. Alternatively, components from the computing device 700 may be combined with other components in a mobile device (not shown), such as a mobile computing device 750. Each of such devices may contain one or more of the computing device 700 and the mobile computing device 750, and an entire system may be made up of multiple computing devices communicating with each other.

The mobile computing device 750 includes a processor 752, a memory 764, an input/output device such as a display 754, a communication interface 766, and a transceiver 768, among other components. The mobile computing device 750 may also be provided with a storage device, such as a micro-drive or other device, to provide additional storage. Each of the processor 752, the memory 764, the display 754, the communication interface 766, and the transceiver 768, are interconnected using various buses, and several of the components may be mounted on a common motherboard or in other manners as appropriate.

The processor 752 can execute instructions within the mobile computing device 750, including instructions stored in the memory 764. The processor 752 may be implemented as a chipset of chips that include separate and multiple analog and digital processors. The processor 752 may provide, for example, for coordination of the other components of the mobile computing device 750, such as control of user interfaces, applications run by the mobile computing device 750, and wireless communication by the mobile computing device 750.

The processor 752 may communicate with a user through a control interface 758 and a display interface 756 coupled to the display 754. The display 754 may be, for example, a TFT (Thin-Film-Transistor Liquid Crystal Display) display or an OLED (Organic Light Emitting Diode) display, or other appropriate display technology. The display interface 756 may comprise appropriate circuitry for driving the display 754 to present graphical and other information to a user. The control interface 758 may receive commands from a user and convert them for submission to the processor 752. In addition, an external interface 762 may provide communication with the processor 752, so as to enable near area communication of the mobile computing device 750 with other devices. The external interface 762 may provide, for example, for wired communication in some implementations, or for wireless communication in other implementations, and multiple interfaces may also be used.

The memory 764 stores information within the mobile computing device 750. The memory 764 can be implemented as one or more of a computer-readable medium or media, a volatile memory unit or units, or a non-volatile memory unit or units. An expansion memory 774 may also be provided and connected to the mobile computing device 750 through an expansion interface 772, which may include, for example, a SIMM (Single In Line Memory Module) card interface. The expansion memory 774 may provide extra storage space for the mobile computing device 750, or may also store applications or other information for the mobile computing device 750. Specifically, the expansion memory 774 may include instructions to carry out or supplement the processes described above, and may include secure information also. Thus, for example, the expansion memory 774 may be provide as a security module for the mobile computing device 750, and may be programmed with instructions that permit secure use of the mobile computing device 750. In addition, secure applications may be provided via the SIMM cards, along with additional information, such as placing identifying information on the SIMM card in a non-hackable manner.

The memory may include, for example, flash memory and/or NVRAM memory (non-volatile random access memory), as discussed below. In some implementations, instructions are stored in an information carrier. In some implementations, the instructions, when executed by one or more processing devices (for example, processor 752), perform one or more methods, such as those described above. The instructions can also be stored by one or more storage devices, such as one or more computer- or machine-readable mediums (for example, the memory 764, the expansion memory 774, or memory on the processor 752). In some implementations, the instructions can be received in a propagated signal, for example, over the transceiver 768 or the external interface 762.

The mobile computing device 750 may communicate wirelessly through the communication interface 766, which may include digital signal processing circuitry where necessary. The communication interface 766 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 768 using a radio-frequency. In addition, short-range communication may occur, such as using a Bluetooth®, Wi-Fi™, or other such transceiver (not shown). In addition, a GPS (Global Positioning System) receiver module 770 may provide additional navigation- and location-related wireless data to the mobile computing device 750, which may be used as appropriate by applications running on the mobile computing device 750.

The mobile computing device 750 may also communicate audibly using an audio codec 760, which may receive spoken information from a user and convert it to usable digital information. The audio codec 760 may likewise generate audible sound for a user, such as through a speaker, e.g., in a handset of the mobile computing device 750. Such sound may include sound from voice telephone calls, may include recorded sound (e.g., voice messages, music files, etc.) and may also include sound generated by applications operating on the mobile computing device 750.

The mobile computing device 750 may be implemented in a number of different forms, as shown in the figure. For example, it may be implemented as a cellular telephone 780. It may also be implemented as part of a smart-phone 782, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

It should also be noted that embodiments of the present disclosure may be provided as one or more computer-readable programs embodied on or in one or more articles of manufacture. The article of manufacture may be any suitable hardware apparatus, such as, for example, a floppy disk, a hard disk, a CD ROM, a CD-RW, a CD-R, a DVD ROM, a DVD-RW, a DVD-R, a flash memory card, a PROM, a RAM, a ROM, or a magnetic tape. In general, the computer-readable programs may be implemented in any programming language. Some examples of languages that may be used include C, C+ +, or Java. The software programs may be further translated into machine language or virtual machine instructions and stored in a program file in that form. The program file may then be stored on or in one or more of the articles of manufacture.

Certain embodiments of the present invention were described above. It is, however, expressly noted that the present invention is not limited to those embodiments, but rather the intention is that additions and modifications to what was expressly described herein are also included within the scope of the invention. Moreover, it is to be understood that the features of the various embodiments described herein were not mutually exclusive and can exist in various combinations and permutations, even if such combinations or permutations were not made express herein, without departing from the scope of the invention. In fact, variations, modifications, and other implementations of what was described herein will occur to those of ordinary skill in the art without departing from the scope of the invention. As such, the invention is not to be defined only by the preceding illustrative description.

In view of the structure, functions and apparatus of the systems and methods described here, in some implementations, systems and methods for creating representations of biological sequences using a draw-ahead utility are provided. Having described certain implementations of methods and apparatus for creating representations of biological sequences using a draw-ahead utility, it will now become apparent to one of skill in the art that other implementations incorporating the concepts of the disclosure may be used. Therefore, the disclosure should not be limited to certain implementations, but rather should be limited only by the scope of the following claims.

## Claims

1. An apparatus for creating a representation of an in-progress biological sequence, the apparatus comprising:
a memory for storing a set of instructions; and
a processor for executing the set of instructions, wherein the instructions, when executed, cause the processor to:
provide a representation of at least a portion of the in-progress biological sequence for presentation on a graphical display;
receive an input corresponding to an amendment to the portion of the in-progress biological sequence;
identify, based at least in part upon a partial match with the amended in-progress biological sequence, one or more biological sequence scaffold candidates selected from an active database of biological sequence scaffold candidates, wherein the one or more biological sequence scaffold candidates comprises a minimum of at least 2 amino acids or at least 10 nucleotides;
provide the one or more biological sequence scaffold candidates for presentation on the graphical display as option(s) for selection by a user for the amendment;
receive an indication of user selection of a first biological sequence scaffold candidate of the one or more biological sequence scaffold candidates provided; and
append the first biological sequence scaffold candidate to the portion of the in-progress biological sequence or replace or partially replace the portion of the in-progress biological sequence with the first biological scaffold candidate, causing the regeneration of the graphical display to include the first biological sequence scaffold candidate in the in-progress biological sequence.

2. The apparatus according to claim 1, wherein the instructions, when executed, further cause the processor to, prior to providing the one or more biological sequence scaffold candidates for presentation, determine that a total number of the one or more biological sequence scaffold candidates does not exceed a threshold number of biological sequence scaffold candidates.

3. The apparatus of any one of the preceding claims, wherein the one or more biological sequence scaffold candidates comprise one or more commonly used biological sequence scaffold candidates; and optionally
wherein the commonly used biological sequence scaffold candidates are scaffolds input and/or selected by a user or group of users at least a threshold number of times.

4. The apparatus according to any one of the preceding claims, wherein the instructions, when executed, cause the processor to arrange the one or more biological sequence scaffold candidates in a ranked order prior to providing the one or more biological sequence scaffold candidates for presentation on the graphical display; and in which case optionally
wherein arranging the one or more biological sequence scaffold candidates in the ranked order comprises:
a) identifying a usage count associated with each biological sequence scaffold candidate of the one or more biological sequence scaffold candidates; or
b) matching a user identifier associated with at least one biological sequence scaffold candidate of the one or more biological sequence scaffold candidates to a user identifier associated with the portion of the in-progress biological sequence.

5. The apparatus according to any one of the preceding claims, wherein receiving the input comprises receiving the input, over a network, from a computing device.

6. The apparatus according to any one of the preceding claims, wherein the one or more biological sequence scaffold candidates are stored in the memory.

7. The apparatus according to any one of the preceding claims, wherein the one or more biological sequence scaffold candidates comprise about 25, 50, 75, 100, 150, 200, or 300 nucleotides, or about 10, 20, 40, 60, 80, or 100 amino acid residues.

8. A non-transitory computer readable medium having instructions stored thereon that, when executed, cause a processor to:
provide a representation of at least a portion of an in-progress biological sequence for presentation on a graphical display;
receive an input corresponding to an amendment to the portion of the in-progress biological sequence;
identify, based at least in part upon a partial match with the amended in-progress biological sequence, one or more biological sequence scaffold candidates selected from an active database of biological sequence scaffold candidates, wherein the one or more biological sequence candidates comprises a minimum of at least 2 amino acids or at least 10 nucleotides; and
provide the one or more biological sequence scaffold candidates for presentation on the graphical display as option(s) for selection by a user for the amendment;
receive an indication of user selection of a first biological sequence scaffold candidate of the one or more biological sequence scaffold candidates provided;
append the first biological sequence scaffold candidate to the portion of the in-progress biological sequence or replace or partially replace the portion of the in-progress biological sequence with the first biological scaffold candidate, causing the regeneration of the graphical display to include the first biological sequence scaffold candidate in the in-progress biological sequence.

9. A method of creating a representation of an in-progress biological sequence, the method comprising:
providing a representation of at least a portion of the in-progress biological sequence for presentation on a graphical display of a user computing device;
receiving an input corresponding to an amendment to the portion of the in-progress biological sequence;
identifying, by a processor of a computing device, based at least in part upon a partial match with the amended in-progress biological sequence, one or more biological sequence scaffold candidates selected from an active database of biological sequence scaffold candidates, wherein the one or more biological sequence candidates comprises a minimum of at least 2 amino acids or at least 10 nucleotides;
providing the one or more biological sequence scaffold candidates for presentation on the graphical display as option(s) for selection by a user for the amendment;
receiving, by the processor, an indication of user selection of a first biological sequence scaffold candidate of the one or more biological sequence scaffold candidates provided; and
appending, by the processor, the first biological sequence scaffold candidate to the portion of the in-progress biological sequence or replace or partially replace the portion of the in-progress biological sequence with the first biological scaffold candidate, causing the regeneration of the graphical display to include the first biological sequence scaffold candidate in the in-progress biological sequence.

10. The method according to claim 9, wherein the user computing device is the computing device.

11. The method according to any one of claims 9 and 10, wherein the amendment comprises addition of at least one of a nucleotide or amino acid residue.

12. The method according to any one of claims 9 to 11, wherein the amendment comprises removal of at least one of a nucleotide or amino acid residue.

## Patentansprüche

1. Vorrichtung zum Erstellen einer Darstellung einer in Bearbeitung befindlichen biologischen Sequenz, wobei die Vorrichtung umfasst:
einen Speicher zum Speichern eines Befehlssatzes; und
einen Prozessor zum Ausführen des Befehlssatzes, wobei die Befehle bei Ausführung den Prozessor veranlassen:
eine Darstellung zumindest eines Teils der in Bearbeitung befindlichen biologischen Sequenz für die Präsentation auf einer grafischen Anzeige bereitzustellen;
eine Eingabe zu empfangen, die einer Änderung an dem Teil der in Bearbeitung befindlichen biologischen Sequenz entspricht;
einen oder mehrere biologische Sequenzgerüst-Kandidaten, der/die aus einer aktiven Datenbank von biologischen Sequenzgerüst-Kandidaten ausgewählt ist/sind, zumindest teilweise auf einer partiellen Übereinstimmung mit der geänderten in Bearbeitung befindlichen biologischen Sequenz basierend zu identifizieren, wobei der eine oder die mehreren biologischen Sequenzgerüst-Kandidaten ein Minimum von wenigstens 2 Aminosäuren oder wenigstens 10 Nukleotiden umfassen;
den einen oder die mehreren biologischen Sequenzgerüst-Kandidaten für die Präsentation auf der grafischen Anzeige als Auswahloption(en) für die Änderung durch einen Benutzer bereitzustellen;
einen Hinweis auf die Benutzerauswahl eines ersten biologischen Sequenzgerüst-Kandidaten aus dem einen oder den mehreren bereitgestellten biologischen Sequenzgerüst-Kandidaten zu empfangen; und
den ersten biologischen Sequenzgerüst-Kandidaten an den Teil der in Bearbeitung befindlichen biologischen Sequenz anzuhängen oder den Teil der in Bearbeitung befindlichen biologischen Sequenz durch den ersten biologischen Sequenzgerüst-Kandidaten zu ersetzen oder teilweise zu ersetzen, wodurch eine Erneuerung der grafischen Anzeige bewirkt wird, so dass diese den ersten biologischen Sequenzgerüst-Kandidaten in der in Bearbeitung befindlichen biologischen Sequenz enthält.

2. Vorrichtung nach Anspruch 1, wobei die Befehle bei Ausführung den Prozessor ferner veranlassen, vor Bereitstellung des einen oder mehreren biologischen Sequenzgerüst-Kandidaten für die Präsentation zu bestimmen, dass eine Gesamtzahl des einen oder der mehreren biologischen Sequenzgerüst-Kandidaten eine Schwellenzahl von biologischen Sequenzgerüst-Kandidaten nicht übersteigt.

3. Vorrichtung nach einem der vorhergehenden Ansprüche, wobei der eine oder die mehreren biologischen Sequenzgerüst-Kandidaten einen oder mehrere häufig verwendete biologische Sequenzgerüst-Kandidaten umfassen; und wobei optional die häufig verwendeten biologischen Sequenzgerüst-Kandidaten Gerüste sind, die von einem Benutzer oder einer Benutzergruppe zumindest mit der Häufigkeit der Schwellenzahl eingegeben und/oder ausgewählt werden.

4. Vorrichtung nach einem der vorhergehenden Ansprüche, wobei die Befehle bei Ausführung den Prozessor veranlassen, den einen oder die mehreren biologischen Sequenzgerüst-Kandidaten in einer Rangfolge anzuordnen, bevor der eine oder die mehreren biologischen Sequenzgerüst-Kandidaten für die Präsentation auf grafischen Anzeige bereitgestellt werden; und wobei in diesem Fall die Anordnung des einen oder der mehreren biologischen Sequenzgerüst-Kandidaten in der Rangfolge optional umfasst:
a) das Ermitteln einer Verwendungshäufigkeit im Zusammenhang mit jedem der biologischen Sequenzgerüst-Kandidaten des einen oder der mehreren biologischen Sequenzgerüst-Kandidaten; oder
b) das Abgleichen einer Benutzerkennung, die zumindest einem biologischen Sequenzgerüst-Kandidaten des einen oder der mehreren biologischen Sequenzgerüst-Kandidaten zugeordnet ist, mit einer Benutzerkennung, die dem Teil der in Bearbeitung befindlichen biologischen Sequenz zugeordnet ist.

5. Vorrichtung nach einem der vorhergehenden Ansprüche, wobei das Empfangen der Eingabe das Empfangen der Eingabe von einer Computervorrichtung über ein Netzwerk umfasst.

6. Vorrichtung nach einem der vorhergehenden Ansprüche, wobei der eine oder die mehreren biologischen Sequenzgerüst-Kandidaten in dem Speicher gespeichert sind.

7. Vorrichtung nach einem der vorhergehenden Ansprüche, wobei der eine oder die mehreren biologischen Sequenzgerüst-Kandidaten etwa 25, 50, 75, 100, 150, 200 oder 300 Nukleotide oder etwa 10, 20, 40, 60, 80 oder 100 Aminosäurereste umfassen.

8. Nichttransitorisches computerlesbares Medium mit darauf gespeicherten Befehlen, die bei Ausführung einen Prozessor veranlassen:
eine Darstellung zumindest eines Teils einer in Bearbeitung befindlichen biologischen Sequenz für die Präsentation auf einer grafischen Anzeige bereitzustellen;
eine Eingabe zu empfangen, die einer Änderung an dem Teil der in Bearbeitung befindlichen biologischen Sequenz entspricht;
einen oder mehrere biologische Sequenzgerüst-Kandidaten, der/die aus einer aktiven Datenbank von biologischen Sequenzgerüst-Kandidaten ausgewählt ist/sind, zumindest teilweise auf einer partiellen Übereinstimmung mit der geänderten in Bearbeitung befindlichen biologischen Sequenz basierend zu identifizieren, wobei der eine oder die mehreren biologischen Sequenzgerüst-Kandidaten ein Minimum von wenigstens 2 Aminosäuren oder wenigstens 10 Nukleotiden umfassen; und
den einen oder die mehreren biologischen Sequenzgerüst-Kandidaten für die Präsentation auf der grafischen Anzeige als Auswahloption(en) für die Änderung durch einen Benutzer bereitzustellen;
einen Hinweis auf die Benutzerauswahl eines ersten biologischen Sequenzgerüst-Kandidaten aus dem einen oder den mehreren bereitgestellten biologischen Sequenzgerüst-Kandidaten zu empfangen; und
den ersten biologischen Sequenzgerüst-Kandidaten an den Teil der in Bearbeitung befindlichen biologischen Sequenz anzuhängen oder den Teil der in Bearbeitung befindlichen biologischen Sequenz durch den ersten biologischen Sequenzgerüst-Kandidaten zu ersetzen oder teilweise zu ersetzen, wodurch eine Erneuerung der grafischen Anzeige bewirkt wird, so dass diese den ersten biologischen Sequenzgerüst-Kandidaten in der in Bearbeitung befindlichen biologischen Sequenz enthält.

9. Verfahren zum Erstellen einer Darstellung einer in Bearbeitung befindlichen biologischen Sequenz, wobei das Verfahren umfasst:
das Bereitstellen einer Darstellung zumindest eines Teils der in Bearbeitung befindlichen biologischen Sequenz für die Präsentation auf einer grafischen Anzeige einer Computervorrichtung eines Benutzers;
das Empfangen einer Eingabe, die einer Änderung an dem Teil der in Bearbeitung befindlichen biologischen Sequenz entspricht;
das Identifizieren eines oder mehrerer biologischer Sequenzgerüst-Kandidaten, der/die aus einer aktiven Datenbank von biologischen Sequenzgerüst-Kandidaten ausgewählt wird/werden, zumindest teilweise basierend auf einer partiellen Übereinstimmung mit der geänderten in Bearbeitung befindlichen biologischen Sequenz durch einen Prozessor, wobei der eine oder die mehreren biologischen Sequenzgerüst-Kandidaten ein Minimum von wenigstens 2 Aminosäuren oder wenigstens 10 Nukleotiden umfassen;
das Bereitstellen des einen oder der mehreren biologischen Sequenzgerüst-Kandidaten für die Präsentation auf der grafischen Anzeige als Auswahloption(en) für die Änderung durch einen Benutzer;
das Empfangen eines Hinweises auf die Benutzerauswahl eines ersten biologischen Sequenzgerüst-Kandidaten aus dem einen oder den mehreren bereitgestellten biologischen Sequenzgerüst-Kandidaten; und
das Anhängen des ersten biologischen Sequenzgerüst-Kandidaten an den Teil der in Bearbeitung befindlichen biologischen Sequenz oder das Ersetzen oder teilweise Ersetzen des Teils der in Bearbeitung befindlichen biologischen Sequenz durch den ersten biologischen Gerüst-Kandidaten, wodurch die Erneuerung der grafischen Anzeige bewirkt wird, so dass diese den ersten biologischen Sequenzgerüst-Kandidaten der in Bearbeitung befindlichen Sequenz enthält.

10. Verfahren nach Anspruch 9, wobei die Benutzer-Computervorrichtung die Computervorrichtung ist.

11. Verfahren nach einem der Ansprüche 9 und 10, wobei die Änderung das Hinzufügen von zumindest einem Nukleotid oder Aminosäurerests umfasst.

12. Verfahren nach einem der Ansprüche 9 bis 11, wobei die Änderung das Entfernen von zumindest einem Nukleotid oder Aminosäurerest umfasst.

## Revendications

1. Appareil pour créer une représentation d'une séquence biologique en cours de développement, appareil comprenant :
- une mémoire pour enregistrer un jeu d'instructions, et
- un processeur pour exécuter le jeu d'instructions,
l'exécution des instructions par le processeur consiste à :
* fournir une représentation d'au moins une partie de la séquence biologique en développement pour la présenter sur un affichage graphique,
* recevoir une entrée correspondant à une modification de la partie de la séquence biologique en développement,
* identifier en fonction d'au moins une partie coïncidant avec la séquence biologique en cours de développement, modifiée, un ou plusieurs candidats d'échafaudage de séquences biologiques choisis dans une base de données active de candidats d'échafaudage de séquences biologiques, ce ou ces candidats d'échafaudage de séquences biologiques ayant au minimum au moins deux acides aminés ou au moins dix nucléotides,
* fournir le ou les candidats d'échafaudage de séquences biologiques pour la présentation sur l'affichage graphique comme option pour être sélectionnés par un utilisateur pour la modification,
* recevoir une indication de sélection d'utilisateur du premier candidat d'échafaudage de séquences biologiques pour un ou plusieurs candidats d'échafaudage de séquences biologiques, et
* relier le premier candidat d'échafaudage de séquences biologiques à la partie de la séquence biologique en développement ou remplacer ou remplacer partiellement la partie de séquences biologiques en développement par le premier candidat d'échafaudage biologique, provoquant la régénération de l'affichage graphique pour inclure le premier candidat d'échafaudage de séquences biologiques dans la séquence biologique en développement.

2. Appareil selon la revendication 1,
dans lequel
les instructions exécutées, font en outre que le processeur, avant de fournir un ou plusieurs candidats d'échafaudage de séquences biologiques pour la présentation, détermine que le nombre total d'un ou plusieurs candidats d'échafaudage de séquences biologiques ne dépasse pas un nombre-seuil de candidats d'échafaudage de séquences biologiques.

3. Appareil selon l'une quelconque des revendications précédentes, selon lequel
le ou les candidats d'échafaudage de séquences biologiques comprennent un ou plusieurs candidats d'échafaudage de séquences biologiques communément utilisés et en option
* les candidats d'échafaudage de séquences biologiques utilisés communément sont des entrées d'échafaudages et/ou sont choisis par un utilisateur ou un groupe d'utilisateurs au moins un nombre-seuil de fois.

4. Appareil selon l'une quelconque des revendications précédentes, selon lequel
les instructions exécutées font que le processeur organise le ou les candidats d'échafaudage de séquences biologiques dans un ordre classé avant de fournir le ou les candidats d'échafaudage de séquences biologiques pour la présentation sur l'affichage graphique et qui, en option :
* arrange le ou les candidats d'échafaudage de séquences biologiques dans un ordre classé consistant à :
a) identifier un comptage d'usage associé à chaque candidat d'échafaudage de séquences biologiques parmi le ou les candidats d'échafaudage de séquences biologiques, ou
b) faire concorder un identifiant d'utilisateur associé à au moins un candidat d'échafaudage de séquences biologiques parmi le ou les candidats d'échafaudage de séquences biologiques pour un identifiant d'utilisateur associé à la partie de la séquence biologique en développement.

5. Appareil selon l'une quelconque des revendications précédentes, selon lequel
recevoir l'entrée consiste à recevoir l'entrée par un réseau à partir d'un calculateur.

6. Appareil selon l'une quelconque des revendications précédentes, selon lequel
le ou les candidats d'échafaudage de séquences biologiques sont enregistrés dans la mémoire.

7. Appareil selon l'une quelconque des revendications précédentes, selon lequel
le ou les candidats d'échafaudage de séquences biologiques ont environ 25, 50, 75, 100, 150, 200, ou 300 nucléotides ou environ 10, 20, 40, 60, 80 ou 100 résidus d'acides aminés.

8. Support non volatile lisible par un ordinateur comportant des instructions enregistrées qui, exécutées font que le processeur :
- fournit une représentation d'au moins une partie d'une séquence biologique en développement pour être présentée sur un affichage graphique,
- reçoit une entrée correspondant à une modification de la partie de la séquence biologique en développement,
- identifie en fonction d'au moins une partie pour une concordance partielle avec la séquence biologique en développement, un ou plusieurs candidats d'échafaudage de séquences biologiques dans une base de données active de candidats d'échafaudage de séquences biologiques, le ou les candidats de séquences biologiques comprenant au minimum au moins 2 acides aminés et au moins 10 nucléotides, et
- fournit le ou les candidats d'échafaudage de séquences biologiques pour être présentés sur l'affichage graphique comme options pour la sélection par l'utilisateur pour faire la modification,
- reçoit une indication de sélection d'utilisateur du premier candidat d'échafaudage de séquences biologiques parmi le ou les candidats d'échafaudage de séquences biologiques fournis,
- accroche le premier candidat d'échafaudage de séquences biologiques à la partie de la séquence biologique en développement ou remplace ou remplace partiellement la partie de la séquence biologique en développement par le premier candidat d'échafaudage biologique de façon à régénérer l'affichage graphique pour inclure le premier candidat d'échafaudage de séquences biologiques dans la séquence biologique en développement.

9. Procédé de création d'une représentation d'une séquence biologique en développement, procédé consistant à :
- fournir une représentation d'au moins une partie de la séquence biologique en développement pour être présentée sur un affichage graphique d'un dispositif de calcul d'utilisateur,
- recevoir une entrée correspondant à une modification de la partie de la séquence biologique en développement,
- identifier par le processeur du dispositif de calcul, en se fondant au moins en partie sur une concordance partielle avec la séquence biologique en développement, modifiée, un ou plusieurs candidats d'échafaudage de séquences biologiques choisis dans une base de données active de candidats d'échafaudage de séquences biologiques, le ou les candidats de séquences biologiques comprenant au moins deux acides aminés ou au moins 10 nucléotides,
- fournir le ou les candidats d'échafaudage de séquences biologiques pour la présentation sur l'affichage graphique comme option pour la sélection par un utilisateur pour faire la modification,
- recevoir le processeur ou l'indication de sélection d'utilisateur du premier candidat d'échafaudage de séquences biologiques parmi le ou les candidats d'échafaudage de séquences biologiques fournis, et
- accrocher par le processeur, le premier candidat d'échafaudage de séquences biologiques à la partie de la séquence biologique en développement ou remplacer ou remplacer partiellement la partie de la séquence biologique en développement par le premier candidat d'échafaudage biologique et produire la régénération de l'affichage graphique pour inclure le premier candidat d'échafaudage de séquences biologiques dans la séquence biologique en développement.

10. Procédé selon la revendication 9,
selon lequel
le dispositif de calcul d'utilisateur est un dispositif de calcul.

11. Procédé selon l'une quelconque des revendications 9 et 10, selon lequel la modification consiste à additionner au moins un nucléotide ou un résidu d'acide aminé

12. Procédé selon l'une quelconque des revendications 9 à 11, selon lequel la modification consiste à enlever au moins un nucléotide ou un résidu d'acide aminé.
